# EUROPÄISCHE PATENTANMELDUNG

(11) **EP 1 529 517 A2**
(43) Veröffentlichungstag der Anmeldung: **11.05.2005**
(21) Anmeldenummer: 04025512.7
(22) Anmeldetag: 27.10.2004
(51) Int. Cl.: A61K 7/13

(54) **Verwendung von Polymeren zur Erhaltung der Farbe in gefärbtem Haar**

(30) Priorität: 04.11.2003 DE 10351352; 02.12.2003 DE 10356255
(71) Anmelder: Clariant GmbH, 65929 Frankfurt am Main (DE)
(72) Erfinder: Henning, Torsten, Dr., 65812 Bad Soben (DE); Löffler, Matthias, Dr., 65527 Niedernhausen (DE); Meder, Markus, Dr., 97956 Werbach (DE)
(74) Vertreter: Paczkowski, Marcus, Dr.

(57) **Zusammenfassung**

Die Erfindung betrifft die Verwendung von einem oder mehreren Polymeren ausgewählt aus Polyethylenglykolen, ethoxylierten quaternären Ammoniumverbindungen und Oligoestern, insbesondere in kosmetischen Formulierungen, zur Stabilisierung der Farbe in gefärbten Keratinfasern, insbesondere in menschlichen Haaren, und zum Schutz dieser Keratinfasern vor Farbverlust.

## Beschreibung

Die Erfindung betrifft die Verwendung von Polymeren zur Stabilisierung der Farbe in gefärbten Keratinfasern, besonders menschlichen Haaren, und zum Schutz vor Farbverlust.

Gefärbte Haare leiden beim Waschen unter Verlust der Farbe. Einige kosmetische Produkte versuchen, dies zu verhindern und die Farbe zu schützen.

EP 509 922 beschreibt die Verwendung von als Thiol funktionalisiertes Polydiorganosiloxan.

WO 94/07455 beschreibt die Verwendung einer Wirkstoffkombination aus alkoxylierten Wollwachsalkoholen mit 1 bis 50 Molekülen Ethylenoxid pro Alkoholmolekül und Pyrrolidoncarbonsäure und/oder -derivat.

DE 197 35 865 beschreibt die Verwendung eines Mittels auf wässriger Basis, enthaltend mindestens eine quaternäre Ammoniumverbindung, Grünen Tee-Extrakt und ein kationisches Polymer.

EP 940 404 beschreibt die Verwendung mindestens eines Organosiloxans, das gegebenenfalls mindestens eine als Antioxidant wirkende Gruppe enthält.

US 6,143,286 beschreibt eine Methode zur Farbstabilisation durch Anwendung einer Komposition, die eine oder mehrere Haarbehandlungswirkstoffe und ein Siloxan mit diund trifunktionellen Einheiten enthält.

Bis dato sind jedoch die auf dem Markt befindlichen Farbschutzshampoos und Haarnachbehandlungsmittel mit Farbschutzfunktion unzureichend. Nach einigen Haarwäschen verliert sich häufig die Haarfarbe trotz Verwendung von Produkten mit Farbschutzanspruch. Zudem sind bekannte Wirkstoffe mit Farbschutz häufig nicht wasserlöslich und führen somit oftmals zu instabilen Formulierungen.

Es bestand daher die Aufgabe, Substanzen zur Verfügung zu stellen, die in der Lage sind, die Farbe in gefärbten Keratinfasern, insbesondere menschlichen Haaren, zu erhalten bzw. einen Schutz von gefärbten Keratinfasern vor Farbverlust zu bewirken und die die obengenannten Nachteile nicht aufweisen.

Überraschenderweise wurde nun gefunden, dass die genannte Aufgabe durch folgende Polymere gelöst wird:
A) Polyethylenglykole der Formel H(OCH₂CH₂)ₙOH mit n gleich 150 bis 900,
B) ethoxylierte quaternäre Ammoniumverbindung der Formel 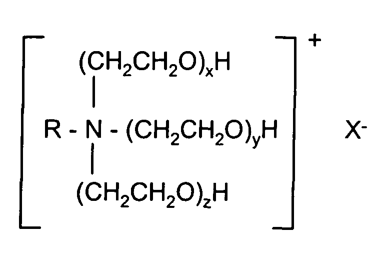 worin R C₄- bis C₃₀-Alkyl bedeutet, x, y und z jeweils unabhängig voneinander eine Zahl von 1 bis 300 bedeuten und X- ein anionisches Gegenion darstellt, und
C) Oligoester, die durch Kondensation von einer oder mehreren Dicarbonsäuren oder deren Estern und einem oder mehreren mehrwertigen Alkoholen, gegebenenfalls in Gegenwart von Anlagerungsprodukten eines Alkylenoxids an Alkohole, Alkylphenole oder Alkylamine, vorzugsweise in Gegenwart von Methylpolyethylenglycol, erhalten werden.

Gegenstand der vorliegenden Erfindung ist daher die Verwendung von einem oder mehreren Polymeren ausgewählt aus
A) Polyethylenglykolen der Formel (1)

   H(OCH₂CH₂)ₙOH (1),

   worin n für eine ganze Zahl zwischen 150 und 900 steht,
B) ethoxylierten quaternären Ammoniumverbindungen der Formel (2) 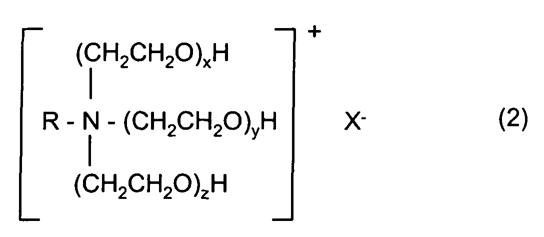 worin
   - R: C₄- bis C₃₀-Alkyl bedeutet,
   - x, y und z: jeweils unabhängig voneinander eine Zahl von 1 bis 300 bedeuten, und
   X⁻ ein anionisches Gegenion darstellt, und
C) Oligoestern, die durch Kondensation von einer oder mehreren Dicarbonsäuren oder deren Estern und einem oder mehreren mehrwertigen Alkoholen, gegebenenfalls in Gegenwart von Anlagerungsprodukten eines Alkylenoxids an Alkohole, Alkylphenole oder Alkylamine, vorzugsweise in Gegenwart von Methylpolyethylenglycol, erhalten werden,

zur Erhaltung der Farbe in gefärbten Keratinfasern, vorzugsweise menschlichem Haar.

Überraschenderweise wurde außerdem gefunden, dass eine Kombination von Polymeren aus zwei oder allen drei beschriebenen Polymertypen in der Lage ist, die Farbe in gefärbten Keratinfasern, besonders menschlichen Haaren, synergistisch besonders gut zu erhalten.

Bei der Verwendung der Polymere zur Erhaltung der Farbe in gefärbten Keratinfasern werden in einer bevorzugten Ausführungsform der Erfindung ein oder mehrere Polyethylenglykole der Formel (1) gemäß Komponente A) und ein oder mehrere ethoxylierte quaternäre Ammoniumverbindungen der Formel (2) gemäß Komponente B) eingesetzt.

Bei der Verwendung der Polymere zur Erhaltung der Farbe in gefärbten Keratinfasern werden in einer weiteren bevorzugten Ausführungsform der Erfindung ein oder mehrere Polyethylenglykole der Formel (1) gemäß Komponente A) und ein oder mehrere Oligoester gemäß Komponente C) eingesetzt.

Bei der Verwendung der Polymere zur Erhaltung der Farbe in gefärbten Keratinfasern werden in einer weiteren Ausführungsform der Erfindung ein oder mehrere ethoxylierte quaternäre Ammoniumverbindungen der Formel (2) gemäß Komponente B) und ein oder mehrere Oligoester gemäß Komponente C) eingesetzt.

Bei der Verwendung der Polymere zur Erhaltung der Farbe in gefärbten Keratinfasern werden in einer weiteren Ausführungsform der Erfindung ein oder mehrere Polyethylenglykole der Formel (1) gemäß Komponente A), ein oder mehrere ethoxylierte quaternäre Ammoniumverbindungen der Formel (2) gemäß Komponente B) und ein oder mehrere Oligoester gemäß Komponente C) eingesetzt.

Vorzugsweise liegen die Polymere bei der erfindungsgemäßen Verwendung in einer kosmetischen Formulierung vor.

Weiterer Gegenstand der Erfindung sind auch kosmetische Formulierungen enthaltend mindestens zwei Polymere ausgewählt aus
A) Polyethylenglykolen der Formel (1)

   H(OCH₂CH₂)ₙOH (1),

   worin n für eine ganze Zahl zwischen 150 und 900 steht,
B) ethoxylierten quaternären Ammoniumverbindungen der Formel (2) 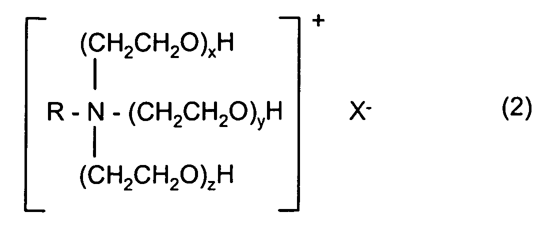 worin
   - R: C₄- bis C₃₀-Alkyl bedeutet,
   - x, y und z: jeweils unabhängig voneinander eine Zahl von 1 bis 300 bedeuten, und
   - X⁻: ein anionisches Gegenion darstellt, und
C) Oligoestern, die durch Kondensation von einer oder mehreren Dicarbonsäuren oder deren Estern und einem oder mehreren mehrwertigen Alkoholen, gegebenenfalls in Gegenwart von Anlagerungsprodukten eines Alkylenoxids an Alkohole, Alkylphenole oder Alkylamine, vorzugsweise in Gegenwart von Methylpolyethylenglycol, erhalten werden.

In einer bevorzugten Ausführungsform der Erfindung enthalten die kosmetischen Formulierungen ein oder mehrere Polyethylenglykole der Formel (1) gemäß Komponente A) und ein oder mehrere ethoxylierte quaternäre Ammoniumverbindungen der Formel (2) gemäß Komponente B).

In einer weiteren bevorzugten Ausführungsform der Erfindung enthalten die kosmetischen Formulierungen ein oder mehrere Polyethylenglykole der Formel (1) gemäß Komponente A) und ein oder mehrere Oligoester gemäß Komponente C).

In einer weiteren bevorzugten Ausführungsform der Erfindung enthalten die kosmetischen Formulierungen ein oder mehrere ethoxylierte quaternäre Ammoniumverbindungen der Formel (2) gemäß Komponente B) und ein oder mehrere Oligoester gemäß Komponente C).

In einer weiteren bevorzugten Ausführungsform der Erfindung enthalten die kosmetischen Formulierungen ein oder mehrere Polyethylenglykole der Formel (1) gemäß Komponente A), ein oder mehrere ethoxylierte quaternäre Ammoniumverbindungen der Formel (2) gemäß Komponente B) und ein oder mehrere Oligoester gemäß Komponente C).

In den Verbindungen der Formel (1) ist n vorzugsweise eine ganze Zahl zwischen 220 und 800.

In den Verbindungen der Formel (2) ist R vorzugsweise C₈- bis C₂₂-Alkyl.

In einer bevorzugten Ausführungsform der Erfindung sind x, y und z jeweils unabhängig voneinander eine Zahl von 1 bis 20, insbesondere eine Zahl von 1 bis 4.

In den Verbindungen der Formel (2) ist die Summe x+y+z eine Zahl von 3 bis 900. In einer weiteren bevorzugten Ausführungsform der Erfindung ist die Summe x+y+z eine Zahl von 4 bis 50, insbesondere eine Zahl von 4 bis 10.

In einer besonders bevorzugten Ausführungsform der Erfindung bedeutet demnach R in Formel (2) C₈-C₂₂-Alkyl und die Summe x+y+z eine Zahl von 4 bis 50, vorzugsweise eine Zahl von 4 bis 10.

In den Verbindungen der Formel (2) ist das anionische Gegenion X- vorzugsweise ausgewählt aus Halogenidanionen, OH- sowie deprotonierten schwachen Säuren, vorzugsweise deprotonierter Milchsäure.

In einer bevorzugten Ausführungsform der Erfindung sind die Oligoester der Komponente C) ausgewählt aus Oligoestern enthaltend Dicarbonsäureeinheiten und Dioleinheiten, vorzugsweise Glykol-, Alkylglykol- und/oder Polyalkylenpolyglykoleinheiten, insbesondere erhalten durch Polykondensation von einer oder mehreren aromatischen Dicarbonsäuren oder deren Estern mit Ethylenglykol und/oder Propylenglykol. Gegebenenfalls können diese Ester auch Polyethylenglykol, Polypropylenglykol, Sulfoisophthalsäure, Sulfobenzoesäure, Isethionsäure, C₁-C₄-Alkohole, oxalkylierte C₁-C₂₄-Alkohole, oxalkylierte C₆-C₁₈-Alkylphenole und/oder oxalkylierte C₈-C₂₄-Alkylamine als Monomere enthalten.

Insbesondere bevorzugt ist die Verwendung von solchen Oligoestern, die erhalten werden durch Polykondensation von
a) 40 bis 52, vorzugsweise 45 bis 50 mol%, einer oder mehrerer Dicarbonsäuren oder deren Estern,
b) 10 bis 60, vorzugsweise 20 bis 35 mol%, Ethylenglykol und/oder Propylenglykol,
c) 3 bis 20, vorzugsweise 0 bis 15 mol%, besonders bevorzugt 10 bis 15 mol%, Polyethylenglykol,
d) 0 bis 10 mol% eines wasserlöslichen Anlagerungsproduktes von 5 bis 80 mol eines Alkylenoxids an 1 mol C₁-C₂₄-Alkohole, C₆-C₁₈-Alkylphenole oder C₈-C₂₄-Alkylamine und
e) 0 bis 10 mol% eines oder mehrerer Polyole mit 3 bis 6 Hydroxylgruppen.

Als Komponente a) zur Herstellung der Copolyester eignen sich beispielsweise Terephthalsäure, Phthalsäure, Isophthalsäure sowie die Mono- und Dialkylester mit C₁-C₆-Alkoholen, wie Dimethylterephthalat, Diethylterephthalat und Di-n-propylterephthalat. Weitere Beispiele für Verbindungen, die als Komponente a) zur Herstellung der Polyester eingesetzt werden können, sind Oxalsäure, Bernsteinsäure, Glutarsäure, Adipinsäure, Fumarsäure, Maleinsäure, Itakonsäure, sowie die Mono- und Dialkylester der Carbonsäuren mit C₁-C₆-Alkoholen, z.B. Oxalsäurediethylester, Bernsteinsäurediethylester, Glutarsäurediethylester, Adipinsäuremethylester, Adipinsäurediethylester, Adipinsäure-di-n-butylester, Fumarsäureethylester und Maleinsäuredimethylester. Sofern die in Betracht kommenden Dicarbonsäuren Anhydride bilden können, sind auch die Anhydride der mindestens 2 Carboxylgruppen aufweisenden Carbonsäuren als Verbindung der Komponente a) zur Herstellung der Oligoester geeignet, z.B. Maleinsäureanhydrid, Phthalsäureanhydrid oder Bernsteinsäureanhydrid. Besonders bevorzugt werden als Verbindung der Komponente a) Terephthalsäure, Phthalsäure, Isophthalsäure sowie deren Dimethyl-, Diethyl-, Dipropyl- und Dibutylester eingesetzt. Es ist selbstverständlich möglich, Mischungen verschiedener Carbonsäuren oder verschiedener Ester einzusetzen. Ebenso kann man auch beispielsweise Mischungen aus Carbonsäuren und Ester oder Mischungen aus Carbonsäuren und Anhydriden bei der Kondensation verwenden.

Als Komponente c) verwendet man Polyethylenglykole mit Molmassen von 500 bis 5000, vorzugsweise von 1000 bis 3000.
Als Komponente d) zur Herstellung der Oligoester kommen wasserlösliche Anlagerungsprodukte von 5 bis 80 mol mindestens eines Alkylenoxids an 1 mol C₁-C₂₄-Alkohole, C₆-C₁₈-Alkylphenole oder C₈-C₂₄-Alkylamine in Betracht. Bevorzugt sind Monomethylether von Polyethylenglykolen. Als Alkylenoxide zur Herstellung der Verbindungen der Komponente d) verwendet man vorzugsweise Ethylenoxid sowie Mischungen aus Ethylenoxid und Propylenoxid. Außerdem eignen sich Mischungen aus Ethylenoxid zusammen mit Propylenoxid und/oder Butylenoxid, Mischungen aus Ethylenoxid, Propylenoxid und Isobutylenoxid oder Mischungen aus Ethylenoxid und mindestens einem Butylenoxid. Diese wasserlöslichen Anlagerungsprodukte der Alkylenoxide sind Tenside. Falls zu ihrer Herstellung Mischungen von Alkylenoxiden verwendet wurden, so können sie die Alkylenoxide in Blöcken oder auch in statistischer Verteilung enthalten.

Geeignete Alkohole, die alkoxyliert werden, sind beispielsweise Octylalkohol, Decylalkohol, Laurylalkohol, Myristylalkohol oder Stearylalkohol, insbesondere aber Methanol, sowie die nach dem Ziegler-Verfahren erhältlichen Alkohole mit 8 bis 24 C-Atomen oder die entsprechenden Oxoalkohole. Von den Alkylphenolen haben insbesondere Octylphenol, Nonylphenol und Dodecylphenol zur Herstellung der entsprechenden Tenside Bedeutung. Von den in Betracht kommenden Alkylaminen verwendet man insbesondere die C₁₂-C₁₈-Monoalkylamine zur Herstellung der wasserlöslichen Tenside.

Als Polyole der Komponente e) kommen beispielsweise in Frage Pentaerythrit, Trimethylolethan, Trimethylolpropan, 1,2,3-Hexantriol, Sorbit, Mannit und Glycerin.

Die Synthese der erfindungsgemäßen Oligoester erfolgt nach an sich bekannten Verfahren, indem die Komponenten a), b) und c) sowie gegebenenfalls d) unter Zusatz eines Katalysators zunächst bei Normaldruck auf Temperaturen von 160 bis ca. 220°C erhitzt werden. Dann wird die Reaktion im Vakuum bei Temperaturen von 160 bis ca. 240°C unter Abdestillieren überschüssiger Glykole fortgesetzt. Für die Reaktion eignen sich die bekannten Umesterungs- und Kondensationskatalysatoren des Standes der Technik, wie beispielsweise Titantetraisopropylat, Dibutylzinnoxid oder Antimontrioxid/Calciumacetat. Bezüglich weiterer Einzelheiten zur Durchführung des Verfahrens wird auf EP 442 101 verwiesen.
Besonders geeignet sind auch die aus EP 241 985 bekannten Polyester, die neben Oxyethylen-Gruppen und Terephthalsäureeinheiten 1,2-Propylen-, 1,2-Butylenund/oder 3-Methoxy-1,2-propylengruppen sowie Glycerineinheiten enthalten und mit C₁-C₄-Alkylgruppen endgruppenverschlossen sind, die in EP 253 567 beschriebenen schmutzablösevermögenden Polymere mit einer Molmasse von 900 bis 9000 g/mol aus Ethylenterephthalat und Polyethylenoxidterephthalat, wobei die Polyethylenglykol-Einheiten Molgewichte von 300 bis 3000 g/mol aufweisen und das Molverhältnis von Ethylenterephthalat zu Polyethylenoxidterephthalat 0,6 bis 0,95 beträgt, und die aus EP 272 033 bekannten, zumindest anteilig durch C₁-C₄-Alkyl- oder Acylreste endgruppenverschlossenen Polyester mit Polypropylenterephthalat- und Polyoxyethylenterephthalat-Einheiten.

Gleichfalls bevorzugt sind Oligoester aus Ethylenterephthalat und Polyethylenoxidterephthalat, in denen die Polyethylenglykol-Einheiten Molgewichte von 750 bis 5000 g/mol aufweisen und das Molverhältnis von Ethylenterephthalat zu Polyethylenoxidterephthalat 50:50 bis 90:10 beträgt und deren Einsatz in Waschmitteln in der deutschen Patentschrift DE 28 57 292 beschrieben ist, sowie Oligoester mit Molgewichten von 15 000 bis 50 000 g/mol aus Ethylenterephthalat und Polyethylenoxidterephthalat, wobei die Polyethylenglykol-Einheiten Molgewichte von 1000 bis 10 000 g/mol aufweisen und das Molverhältnis von Ethylenterephthalat zu Polyethylenoxidterephthalat 2:1 bis 6:1 beträgt, die gemäß DE 33 24 258 in Waschmitteln eingesetzt werden können.

Ebenfalls bevorzugt sind die in DE 19 644 034 beschriebenen Oligoester der Formel 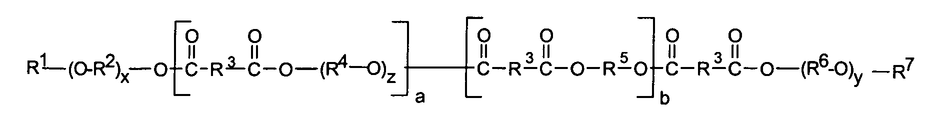 worin
- R¹ und R⁷: lineares oder verzweigtes C₁-C₁₈-Alkyl,
- R² und R⁶: Ethylen,
- R³: 1,4-Phenylen,
- R⁴: Ethylen,
- R⁵: Ethylen, 1,2-Propylen oder statistische Gemische von beliebiger Zusammensetzung von eiden,
- x und y: unabhängig voneinander eine Zahl zwischen 1 und 500,
- z: eine Zahl zwischen 10 und 140,
- a: eine Zahl zwischen 1 und 12,
- b: eine Zahl zwischen 7 und 40,
bedeuten, wobei a+b mindestens gleich 11 ist.

Bevorzugt bedeuten unabhängig voneinander
- R¹ und R⁷: lineares oder verzweigtes C₁-C₄-Alkyl,
- x und y: eine Zahl zwischen 3 und 45,
- z: eine Zahl zwischen 18 und 70,
- a: eine Zahl zwischen 2 und 5,
- b: eine Zahl zwischen 8 und 12,
- a+b: eine Zahl zwischen 12 und 18 oder zwischen 25 und 35.

Die in DE 19 644 034 beschriebenen Oligoester werden aus Dimethylterephthalat, Ethylen- und/oder Propylenglykol, Polyethylenglykol und C₁- bis C₁₈-Alkylpolyethylenglykol unter Zusatz eines Katalysators zunächst durch Umesterung bei Temperaturen von 160 bis ca. 220°C und destillativer Abtrennung des Methanols bei Normaldruck und anschließender destillativer Abtrennung der überschüssigen Glykole bei Temperaturen von 160 bis ca. 240°C erhalten.

Vorzugsweise enthalten die kosmetischen Formulierungen, bezogen auf die fertigen Formulierungen, 0,1 bis 50 Gew.-%, besonders bevorzugt 0,5 bis 10 Gew.-%, insbesondere bevorzugt 1 bis 4 Gew.-% der Polymere.

Die Verbindungen der Formel (1) sind vorzugsweise von 0,1 bis 10 Gew.-%, bezogen auf die fertige kosmetische Formulierung, in der kosmetischen Formulierung enthalten.

Die Verbindungen der Formel (2) sind vorzugsweise von 0,1 bis 10 Gew.-%, bezogen auf die fertige kosmetische Formulierung, in der kosmetischen Formulierung enthalten.

Die Oligoester der Komponente C) sind vorzugsweise von 0,1 bis 10 Gew.-%, bezogen auf die fertige kosmetische Formulierung, in der kosmetischen Formulierung enthalten.

Bei den kosmetischen Formulierungen handelt es sich bevorzugt um Shampoos, insbesondere Haarshampoos, Spülungen, Conditioner, Cremespülungen, Gele, insbesondere Haargele und sonstige Haarstylingprodukte.

Dabei beträgt die Gesamtmenge an Tensiden, bezogen auf die fertigen kosmetischen Formulierungen, bevorzugt zwischen 5 und 70 Gew.-%, besonders bevorzugt zwischen 10 und 40 Gew.-%, insbesondere bevorzugt zwischen 12 und 35 Gew.-%.

Als anionische Tenside sind bevorzugt (C₁₀-C₂₀)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfat, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäure-methyltauride, Fettsäuresarkosinate, Sulforicinoleate, Amphoacetate- oder -glycinate und/oder Acylglutamate. Die anionischen Tenside werden bevorzugt in Form ihrer wasserlöslichen oder in Wasser dispergierbaren Salze eingesetzt, z.B. als Natrium-, Kalium-, Magnesium-, Ammonium-, Mono-, Di- und Triethanolammonium- oder Alkylammonium-Salze.
Der Gewichtsanteil der anionischen Tenside beträgt, bezogen auf die fertigen kosmetischen Formulierungen, bevorzugt 0,1 bis 50 Gew.-%, besonders bevorzugt 7 bis 30 Gew.-%, insbesondere besonders bevorzugt 9 bis 18 Gew.-%.

Als kationische Tenside bevorzugt sind quartäre Ammonium-Salze, bevorzugt Di-(C₁₀-C₂₄)Alkyl-dimethyl-ammonium-chlorid und -bromid, besonders bevorzugt Di-(C₁₂-C₁₈)Alkyl-dimethyl-ammonium-chlorid und -bromid; (C₁₀-C₂₄)Alkyl-dimethylethylammonium-chlorid und -bromid; (C₁₀-C₂₄)Alkyl-trimethyl-ammonium-chlorid und -bromid, bevorzugt Cetyl-trimethyl-ammonium-chlorid und -bromid und (C₂₀-C₂₂)Alkyl-trimethyl-ammonium-chlorid und -bromid; (C₁₀-C₂₄)Alkyl-dimethylbenzyl-ammonium-chlorid und -bromid, bevorzugt (C₁₂-C₁₈)-Alkyl-dimethylbenzyl-ammonium-chlorid; N-(C₁₀-C₁₈)Alkyl-pyridinium-chlorid und -bromid, bevorzugt N-(C₁₂-C₁₆)Alkyl-pyridinium-chlorid und -bromid; N-(C₁₀-C₁₈)Alkyl-isochinolinium-chlorid, -bromid und -monoalkylsulfat; N-(C₁₂-C₁₈)-Alkylpolyoylaminoformylmethyl-pyridiniumchlorid; N-(C₁₂-C₁₈)Alkyl-N-methyl-morpholinium-chlorid, -bromid und -monoalkylsulfat; N-(C₁₂-C₁₈)Alkyl-N-ethyl-morpholinium-chlorid, -bromid und -monoalkylsulfat; (C₁₆-C₁₈)Alkyl-pentaoxethyl-ammonium-chlorid; Diisobutylphenoxyethoxyethyldimethylbenzylammonium-chlorid; Salze des N,N-Diethylaminoethylstearylamids und -oleylamids mit Salzsäure, Essigsäure, Milchsäure, Zitronensäure und Phosphorsäure; N-Acyl-aminoethyl-N,N-diethyl-N-methyl-ammoniumchlorid, -bromid und -monoalkylsulfat; und/oder N-Acylaminoethyl-N,N-diethyl-N-benzyl-ammonium-chlorid, -bromid und -monoalkylsulfat, wobei es sich bei den Acylresten vorzugsweise um Stearoyl - oder Oleoylreste handelt.

Der Gewichtsanteil der kationischen Tenside, bezogen auf die fertigen kosmetischen Formulierungen beträgt bevorzugt 1 bis 10 Gew.-%, besonders bevorzugt 2 bis 7 Gew.-% und insbesondere bevorzugt 3 bis 5 Gew.-%.

Als nichtionische Tenside bevorzugt sind Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (® Pluronics); Fettsäureamidpolyethylenglykole; N-Alkyl-, N-Alkoxypolyhydroxyfettsäureamid, bevorzugt Fettsäure-N-methylglucamide und Saccharoseester; Polyglykolether; Alkylpolyglycoside; und/oder Phosphorsäureester (Mono-, Di- und Triphosphorsäureester ethoxyliert und nicht-ethoxyliert).

Der Gewichtsanteil der nichtionischen Tenside, bezogen auf die fertigen kosmetischen Formulierungen, beträgt bevorzugt 1 bis 20 Gew.-%, besonders bevorzugt 2 bis 10 Gew.-%, insbesondere bevorzugt 3 bis 7 Gew.-%.

Als Amphotenside bevorzugt sind N-(C₁₂-C₁₈)Alkyl-β-aminopropionate und N-(C₁₂-C₁₈)Alkyl-β-iminodipropionate als Alkali- und Mono-, Di- und Trialkylammonium-Salze; N-Acylaminoalkyl-N,N-dimethyl-acetobetain, bevorzugt N-(C₈-C₁₈)Acyl-aminopropyl-N,N-dimethylacetobetain; (C₁₂-C₁₈)-Alkyl-dimethylsulfopropyl-betain; Amphotenside auf Basis Imidazolin (Handelsnamen Miranol®, Steinapon®), bevorzugt das Natrium-Salz des 1-(β-Carboxy-methyloxyethyl)-1-(carboxymethyl)-2-lauryl-imidazoliniums; Aminoxide, z.B. (C₁₂-C₁₈)Alkyl-dimethylaminoxide; und/oder Fettsäureamidoalkyl-dimethylaminoxide.

Der Gewichtsanteil der amphoteren Tenside beträgt, bezogen auf die fertigen kosmetischen Formulierungen, bevorzugt 0,5 bis 20 Gew.-% und besonders bevorzugt 1 bis 10 Gew.-%.

Des weiteren können in den kosmetischen Formulierungen schaumverstärkende Co-Tenside aus der Gruppe Alkylbetaine, Alkylamidobetaine, Aminopropionate, Aminoglycinate, Imidazoliniumbetaine, Sulfobetaine, Aminoxide, Fettsäurealkanolamide und Polyhydroxyamide eingesetzt werden.

Besonders bevorzugte Tenside sind Laurylsulfat, Laurethsulfat, Cocoamidipropylbetain, Natriumcocoylglutamat, Di-natriumlaurethsulfosuccinat und/oder Cocosfettsäurediethanolamid.

Weiterhin können die kosmetischen Formulierungen Überfettungsmittel, Fette, Wachse, Stabilisatoren, biogene Wirkstoffe, Glycerin, Konservierungsmittel, Perlglanzmittel, Farb- und Duftstoffe, Lösungsmittel, Trübungsmittel, Verdickungsmittel, Dispergiermittel, Eiweißderivate (z.B. Gelatine), Collagenhydrolysate, Polypeptide auf natürlicher und synthetischer Basis, Eigelb, Lecithin, Lanolin, Lanolinderivate, Fettalkohole, Silicone, deodorierende Mittel, Stoffe mit keratolytischer und keratoplastischer Wirkung, Enzyme, Trägersubstanzen, feuchtigkeitsspendende Stoffe und/oder antimikrobiell wirkende Agentien enthalten.

Als Überfettungsmittel bevorzugt sind polyethoxylierte Lanolinderivate, Lecithinderivate, Polyolfettsäureester, Monoglyceride und Fettsäurealkanolamide, wobei letztere gleichzeitig als Schaumstabilisatoren geeignet sind.

Bevorzugte als Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol, in Frage.

Als Stabilisatoren können Metallsalze von Fettsäuren, wie z.B. Magnesium-, Aluminiumund/oder Zinkstearat, eingesetzt werden.

Unter biogenen Wirkstoffen sind beispielsweise Pflanzenextrakte und Vitaminkomplexe zu verstehen.

Als Konservierungsmittel eignen sich beispielsweise Phenoxyethanol, Formaldehydabspalter, Parabene, Pentandiol und Sorbinsäure.

Als Farbstoffe können die für kosmetische Zwecke geeigneten und zugelassenen Substanzen verwendet werden.

Als Verdickungs- und Dispergiermittel bevorzugt sind Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate, sowie natürliche Gummen, Carboxyvinylpolymere (z.B. die Carbopol®-Typen 934, -940, -941,-956,-980,-981, -1342 und -1382). Als Verdickungs- und Dispergiermittel besonders geeignet sind Ethylenglycolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl-(C₁₂-C₂₂)-, bevorzugt (C₁₆-C₁₈),-amidobenzoesäure und deren lösliche Salze und/oder N,N-di(C₁₆-C₁₈)-amidobenzoesäure und deren Derivate.

Die Verdickungs- und Dispergiermittel werden, bezogen auf die fertigen kosmetischen Formulierungen, vorzugsweise von 0,5 bis 10 Gew.-%, besonders bevorzugt von 0,5 bis 5 Gew.-% und insbesondere bevorzugt von 1 bis 4 Gew.-% eingesetzt.

Die gewünschte Viskosität der kosmetischen Formulierungen kann durch Zugabe von Wasser und/oder organischen Lösungsmitteln oder durch Zugabe einer Kombination aus organischen Lösungsmitteln und Verdickungsmitteln eingestellt werden.

Als organische Lösungsmittel kommen prinzipiell alle ein- oder mehrwertigen Alkohole und ethoxylierten Alkohole in Betracht. Bevorzugt werden Alkohole mit 1 bis 4 Kohlenstoffatomen, wie Ethanol, Propanol, Isopropanol, n-Butanol und Iso-Butanol, Glycerin und Mischungen aus den genannten Alkoholen. Weitere geeignete Lösungsmittel sind beispielsweise Triacetin (Glycerintriacetat) und 1-Methoxy-2-propanol.

Die kosmetischen Formulierungen enthalten, bezogen auf die fertigen Formulierungen, die Alkohole vorzugsweise in Gewichtsmengen von 0,1 bis 50 Gew.-%.

Als Trägermaterialien eignen sich bevorzugt pflanzliche Öle, natürliche und gehärtete Öle, Wachse, Fette, Wasser, Alkohole, Polyole, Glycerol, Glyceride, flüssige Paraffine, flüssige Fettalkohole, Sterol, Cellulose und Cellulose-Derivate.

Als antifungizide Wirkstoffe können Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Oczopyrox eingesetzt werden.

Als weitere pflegende Stoffe kommen Allantoin und Bisabolol in Frage, bevorzugt in Gewichtsmengen von 0,0001 bis 10 Gew.-%.

Als kationische Polymere bevorzugt sind kationische Cellulosederivate, kationische Stärke, Copolymere von Diallylammoniumsalzen und Acrylamiden, quaternierte Vinylpyrrolidon/ Vinylimidazol-Polymere, Kondensationsprodukte von Polyglycolen und Aminen, quaternierte Kollagenpolypeptide, quaternierte Weizenpolypeptide, Polyethylenimin, kationische Siliconpolymere, wie z.B. Amidomethicone, Copolymere der Adipinsäure und Dimethylaminohydroxypropyldiethylentriamin, Polyaminopolyamid und kationische Chitinderivate, wie beispielsweise Chitosan.
Geeignete Siliconverbindungen sind beispielsweise Dimethylpolysiloxan, Methylphenylpolysiloxane, cyclische Silicone sowie amino-, fettsäure-, alkohol-, polyether-, epoyx-, fluor- und/ oder alkylmodifizierte Siliconverbindungen, sowie Polyalkylsiloxane, Polyalkylarylsiloxane, Polyethersiloxan-Copolymere, wie in US 5,104,645 und den darin zitierten Schriften beschrieben, die bei Raumtemperatur sowohl flüssig als auch harzförmig vorliegen können.

Die kosmetischen Formulierungen können mit konventionellen Ceramiden, Pseudoceramiden, Fettsäure-N-alkylpolyhydroxyalkylamide Cholesterin, Cholesterinfettsäureestern, Fettsäuren, Triglyceriden, Cerebrosiden, Phospholipiden und ähnlichen Stoffen, abgemischt werden.

Als perlglanzgebende Verbindungen geeignet sind Fettsäuremonoalkanolamide; Fettsäuredialkanolamide; Monoester und Diester von Alkylenglycol, insbesondere solche aus Ethylenglykol, Propylenglycol oder deren Oligomeren und höheren Fettsäuren, z.B. Palmitinsäure, Stearinsäure, Behensäure oder Mischungen davon; Mono- oder Diester von Alkylenglykolen mit Fettsäuren; Fettsäuren und deren Metallsalze; Monoester oder Polyester von Glycerin mit Carbonsäuren; und/oder Ketosulfone. Als perlglanzgebende Komponente besonders bevorzugt sind Ethylenglycoldistearat und Polyethylenglycoldistearat mit 3 Glykoleinheiten.

Als feuchtigkeitsspendende Substanz bevorzugt sind Isopropylpalmitat, Glycerin und/oder Sorbitol. Sie liegen, bezogen auf die fertigen Formulierungen, vorzugsweise in Gewichtsmengen von 0,1 bis 50 Gew.-% vor.

Die nachfolgenden Beispiele sollen den Gegenstand der Erfindung näher erläutern, ohne ihn darauf einzuschränken.

### Beispiele

Strähnen von gebleichtem blonden Haar wurden mit je 2 ml für 30 Minuten mit einer handelsüblichen Farbe (Wella Viva Pure red) gefärbt. L und E Werte im CILAB System wurden mit einem Spektrophotometer (Datacolor Mercury 2000) bestimmt. Hierbei geben die L Werte ein Maß für die Helligkeit und die E Werte ein Maß für den Farbton an. Anschließend wurden die Strähnen vier Mal für jeweils eine Minute mit 0,5 ml Standard- oder Testshampoo gewaschen und jeweils 30 Sekunden ausgespült. Anschließend wurden L und E Werte gemessen und die Differenzen als DL und DE bestimmt.

| Rezeptur: Standard | Testformulierungen | | | | |
|---|---|---|---|---|---|
| | S | A | B | C | D |
| Sodium Laureth Sulfate (27 % aktiv) | 48,1 | 48,1 | 48,1 | 48,1 | 48,1 |
| Cocamidopropyl Betaine (30 % aktiv) | 6,7 | 6,7 | 6,7 | 6,7 | 6,7 |
| Polyglykol 35000 S | - | 1,0 | - | - | 0,3 |
| Genamin KSL | - | - | 1,0 | - | 0,3 |
| Aristoflex PEA | - | - | - | 1,0 | 0,3 |
| E-Wasser | ad 100 | ad 100 | ad 100 | ad 100 | ad 100 |
| NaCl | 1,8 | 1,8 | 1,8 | 1,8 | 1,8 |

Ergebnisse der Farbmessungen (Angabe in Reflexionseinheiten):

| | S | A | B | C | D |
|---|---|---|---|---|---|
| DL | 4,0 | 1,2 | 2,5 | 1,5 | 1,1 |
| DE | 7,5 | 3,5 | 3,5 | 4,1 | 3,1 |

Eine deutliche Wirksamkeit der einzelnen Wirkstoffe sowie der Kombination aller drei Wirkstoffe ist zu sehen.

| Formulierungsbeispiel 1: Haarshampoo | | |
|---|---|---|
| Komponenten | | Menge in Gew.-% |
| 1 | Genapol LRO liquid | 11,1 |
| 2 | Duftstoff | 0,3 |
| 3 | E-Wasser | ad 100 |
| 4 | Genagen KB | 12,0 |
| 5 | Genagen LAA | 11,6 |
| 6 | Polyglykol 35000 S | 1,0 |
| | | |

| Komponenten | | Menge in Gew.-% |
|---|---|---|
| 7 | Aristoflex PEA | 1,0 |
| 8 | Zitronensäure (50 %ig in Wasser) | 1,2 |
| 9 | Farbstofflösung | q.s. |
| 10 | Konservierungsmittel | q.s. |

### Herstellung:

Die Komponente 1 wurde vorgelegt, anschließend wurden die anderen Komponenten in der angegebenen Reihenfolge eingerührt.

| Formulierungsbeispiel 2: Cremespülung | | |
|---|---|---|
| Komponenten | | Menge in Gew.-% |
| 1 | Genamin DSAC | 1,5 |
| 2 | Hostacerin T-3 | 1,5 |
| 3 | Cetylalkohol | 2,5 |
| 4 | Paraffinöl, hochviskos | 1,0 |
| 5 | Genamin KSL | 2,0 |
| 6 | E-Wasser | ad 100 |
| 7 | Polyglykol 20000 S | 1,0 |
| 8 | Konservierungsmittel | q.s. |
| 9 | Parfümöl | 0,3 |
| 10 | Farbstofflösung | q.s. |
| 11 | Zitronensäure | q.s. |

### Herstellung:

Die Komponenten 1 bis 4 wurden bei ca. 75°C aufgeschmolzen. Anschließend wurden die Komponenten 5 bis 8 auf ca. 75°C erwärmt und unter Rühren zugesetzt. Anschließend wurde kaltgerührt. Danach wurden bei ca. 35°C die Komponenten 9 und 10 eingerührt und schließlich wurde der pH-Wert mit Zitronensäure auf pH 4 eingestellt.

| Verzeichnis der eingesetzten Produkte: | |
|---|---|
| Polyglykol 20000 S (Clariant GmbH) | PEG-450, Polyethylenglykol mit mittlerer Molmasse 20000 g/mol |
| Polyglykol 35000 S (Clariant GmbH) | PEG-800, Polyethylenglykol mit mittlerer Molmasse 35000 g/mol |
| Genagen® LAA (Clariant GmbH) | Laurylamphoacetat, Na-Salz |
| Genagen® KB (Clariant GmbH) | Cocoylbetain |
| Genapol® LRO (Clariant GmbH) | Sodium Laureth Sulfate |
| Genamin® KSL (Clariant GmbH) | PEG-5 Stearyl Ammonium Lactate |
| Hostacerin® T-3 (Clariant GmbH) | Ceteareth-3 |
| Genamin® DSAC (Clariant GmbH) | Distearyldimonium Chloride |
| Aristoflex® PEA (Clariant GmbH) | Polypropylene Terephthalate |

## Patentansprüche

1. Verwendung von einem oder mehreren Polymeren ausgewählt aus
A) Polyethylenglykolen der Formel (1)
H(OCH₂CH₂)ₙOH (1),
worin n für eine ganze Zahl zwischen 150 und 900 steht,
B) ethoxylierten quaternären Ammoniumverbindungen der Formel (2) 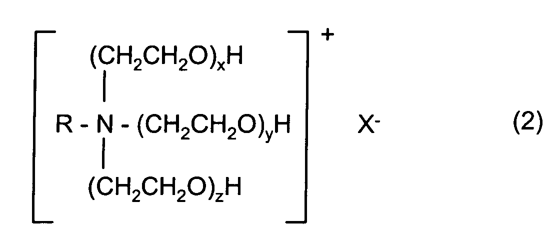 worin
R C₄- bis C₃₀-Alkyl bedeutet,
x, y und z jeweils unabhängig voneinander eine Zahl von 1 bis 300 bedeuten, und
X⁻ ein anionisches Gegenion darstellt, und
C) Oligoestern, die durch Kondensation von einer oder mehreren Dicarbonsäuren oder deren Estern und einem oder mehreren mehrwertigen Alkoholen, gegebenenfalls in Gegenwart von Anlagerungsprodukten eines Alkylenoxids an Alkohole, Alkylphenole oder Alkylamine, vorzugsweise in Gegenwart von Methylpolyethylenglycol, erhalten werden,
zur Erhaltung der Farbe in gefärbten Keratinfasern.

2. Verwendung nach Anspruch 1, **dadurch gekennzeichnet, dass** es sich bei den Keratinfasern um menschliches Haar handelt.

3. Verwendung nach Anspruch 1 oder 2, **dadurch gekennzeichnet, dass** die Polymere in einer kosmetischen Formulierung vorliegen.

4. Verwendung nach einem oder mehreren der Ansprüche 1 bis 3, **dadurch gekennzeichnet, dass** n in Formel (1) für eine ganze Zahl zwischen 220 und 800 steht.

5. Verwendung nach einem oder mehreren der Ansprüche 1 bis 4, **dadurch gekennzeichnet, dass** die kosmetische Formulierung 0,1 bis 10 Gew.-% der Verbindungen der Formel (1), bezogen auf die fertige kosmetische Formulierung, enthält.

6. Verwendung nach einem oder mehreren der Ansprüche 1 bis 5, **dadurch gekennzeichnet, dass** R in Formel (2) C₈- bis C₂₂-Alkyl bedeutet und die Summe x+y+z eine Zahl von 4 bis 50 ist.

7. Verwendung nach einem oder mehreren der Ansprüche 1 bis 6, **dadurch gekennzeichnet, dass** das anionische Gegenion X- in Formel (2) ausgewählt ist aus Halogenidanionen, OH- sowie deprotonierten schwachen Säuren, vorzugsweise deprotonierter Milchsäure.

8. Verwendung nach einem oder mehreren der Ansprüche 1 bis 7, **dadurch gekennzeichnet, dass** die kosmetische Formulierung 0,1 bis 10 Gew.-% der Verbindungen der Formel (2), bezogen auf die fertige kosmetische Formulierung, enthält.

9. Verwendung nach einem oder mehreren der Ansprüche 1 bis 8, **dadurch gekennzeichnet, dass** die Oligoester der Komponente C) ausgewählt sind aus Verbindungen, die durch Polykondensation von
a) 40 bis 52, vorzugsweise 45 bis 50 mol%, einer oder mehrerer aromatischer Dicarbonsäuren oder deren Ester,
b) 10 bis 60, vorzugsweise 20 bis 35 mol%, Ethylenglykol und/oder Propylenglykol,
c) 0 bis 20, vorzugsweise 0 bis 15 mol%, besonders bevorzugt 10 bis 15 mol%, Polyethylenglykol,
d) 0 bis 10 mol% eines wasserlöslichen Anlagerungsproduktes von 5 bis 80 mol eines Alkylenoxids an 1 mol C₁-C₂₄-Alkohole, C₆-C₁₈-Alkylphenole oder C₈-C₂₄-Alkylamine und
e) 0 bis 10 mol% eines oder mehrerer Polyole mit 3 bis 6 Hydroxylgruppen erhalten werden.

10. Verwendung nach einem oder mehreren der Ansprüche 1 bis 9, **dadurch gekennzeichnet, dass** die kosmetische Formulierung 0,1 bis 10 Gew.-% Oligoester der Komponente C), bezogen auf die fertige kosmetische Formulierung, enthält.

11. Verwendung nach einem oder mehreren der Ansprüche 1 bis 10, **dadurch gekennzeichnet, dass** ein oder mehrere Polyethylenglykole der Formel (1) gemäß Komponente A) und ein oder mehrere ethoxylierte quaternäre Ammoniumverbindungen der Formel (2) gemäß Komponente B) eingesetzt werden.

12. Verwendung nach einem oder mehreren der Ansprüche 1 bis 10, **dadurch gekennzeichnet, dass** ein oder mehrere Polyethylenglykole der Formel (1) gemäß Komponente A) und ein oder mehrere Oligoester gemäß Komponente C) eingesetzt werden.

13. Verwendung nach einem oder mehreren der Ansprüche 1 bis 10, **dadurch gekennzeichnet, dass** ein oder mehrere ethoxylierte quaternäre Ammoniumverbindungen der Formel (2) gemäß Komponente B) und ein oder mehrere Oligoester gemäß Komponente C) eingesetzt werden.

14. Verwendung nach einem oder mehreren der Ansprüche 1 bis 10, **dadurch gekennzeichnet, dass** ein oder mehrere Polyethylenglykole der Formel (1) gemäß Komponente A), ein oder mehrere ethoxylierte quaternäre Ammoniumverbindungen der Formel (2) gemäß Komponente B) und ein oder mehrere Oligoester gemäß Komponente C) eingesetzt werden.

15. Kosmetische Formulierung enthaltend mindestens zwei Polymere ausgewählt aus
A) Polyethylenglykolen der Formel (1)
H(OCH₂CH₂)ₙOH (1),
worin n für eine ganze Zahl zwischen 150 und 900 steht,
B) ethoxylierten quaternären Ammoniumverbindungen der Formel (2) 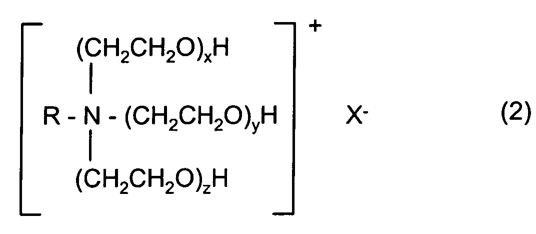 worin
R C₄- bis C₃₀-Alkyl bedeutet,
x, y und z jeweils unabhängig voneinander eine Zahl von 1 bis 300 bedeuten, und
X⁻ ein anionisches Gegenion darstellt, und
C) Oligoestern, die durch Kondensation von einer oder mehreren Dicarbonsäuren oder deren Estern und einem oder mehreren mehrwertigen Alkoholen, gegebenenfalls in Gegenwart von Anlagerungsprodukten eines Alkylenoxids an Alkohole, Alkylphenole oder Alkylamine, vorzugsweise in Gegenwart von Methylpolyethylenglycol, erhalten werden.

16. Kosmetische Formulierung gemäß Anspruch 15, **dadurch gekennzeichnet, dass** sie ein oder mehrere Polyethylenglykole der Formel (1) gemäß Komponente A) und ein oder mehrere ethoxylierte quaternäre Ammoniumverbindungen der Formel (2) gemäß Komponente B) enthält.

17. Kosmetische Formulierung gemäß Anspruch 15, **dadurch gekennzeichnet, dass** sie ein oder mehrere Polyethylenglykole der Formel (1) gemäß Komponente A) und ein oder mehrere Oligoester gemäß Komponente C) enthält.

18. Kosmetische Formulierung gemäß Anspruch 15, **dadurch gekennzeichnet, dass** sie ein oder mehrere ethoxylierte quaternäre Ammoniumverbindungen der Formel (2) gemäß Komponente B) und ein oder mehrere Oligoester gemäß Komponente C) enthält.

19. Kosmetische Formulierung gemäß Anspruch 15, **dadurch gekennzeichnet, dass** sie ein oder mehrere Polyethylenglykole der Formel (1) gemäß Komponente A), ein oder mehrere ethoxylierte quaternäre Ammoniumverbindungen der Formel (2) gemäß Komponente B) und ein oder mehrere Oligoester gemäß Komponente C) enthält.
